# EUROPEAN PATENT SPECIFICATION

(11) **EP 1 971 388 B1**
(45) Date of publication and mention of the grant of the patent: **17.08.2016**
(21) Application number: 06845089.9
(22) Date of filing: 12.12.2006
(51) Int. Cl.: A61B 17/34

(54) **CLEAR VIEW CANNULA**
KLARSICHTKANÜLE
CANULE AVEC UNE BONNE VISIBILITÉ

(30) Priority: 10.01.2006 US 328660
(43) Date of publication of application: 24.09.2008
(73) Proprietor: Lyon, Thomas R., Brooklyn, NY 11215 (US)
(72) Inventor: Lyon, Thomas R., Brooklyn, NY 11215 (US)
(74) Representative: Lamb, Martin John Carstairs
(86) International application number: PCT/US2006/046998
(87) International publication number: WO 2007/081468

(56) References cited:
- EP-A1- 0 589 452
- WO-A2-2005/037079
- US-A- 4 535 773
- US-A- 5 232 451
- US-A- 5 860 997
- US-A1- 2005 085 771
- US-A1- 2005 085 771

## Description

### FIELD OF THE INVENTION

The present invention relates to cannulas and more specifically to cannulas used in arthroscopic minimally invasive surgery.

### BACKGROUND OF THE INVENTION

Cannulas are surgical instruments that are used in minimally invasive surgery to introduce auxiliary surgical instruments into a body of a patient. Many cannulas have specialized structures that can expand after penetration into a body cavity. The expandable portion provides functions such as anchoring the cannula within the body and enhancing visibility into the patient.

The control of the cannula during and after the initial penetration is an important part of any surgical procedure. The deployment of the expandable portion is frequently performed by manually forcing an outer cannula distally relative to an inner cannula. One such application is U.S. Pat. No. 6,632,197 to Lyon. The use of this type of cannula may be limited by the surgeon's inability to determine the exact positional relationship of the tubes of the cannula during the surgical procedure. An additional problem is that the cannulas lack the lateral support necessary to be free standing and therefore often need to be manually supported at a desired angular position relative to the patient's body during the surgical procedure.

A cannula is needed that has an enhanced system for the control of the movement of the tubes of the cannula and retention of the cannula in an angular position relative to the patient. According to the present disclosure, a cannula is provided which has a uniquely enhanced system for the control of the positioning and limiting of the tubes of the cannula as well as the retaining of the angular position of the cannula during surgical procedures. This enhanced system for control is beyond that of present systems and thus permits a controlled deployment, use and retraction of the cannula that heretofore has been unachievable in minimally invasive medical instruments.

US5232451 discloses a cannula for use in surgical operations according to the preamble of Claim 1.

### SUMMARY OF THE INVENTION

The present invention provides a clear view cannula for use by a single hand in arthroscopic surgical operations as set out in Claim 1. A plurality of flexibly movable shield members are positioned on the distal end portion of one of the tubes. A counter-pressure ring is connected to the second tube by engagement means that secure the counter-pressure ring at predetermined positions along the longitudinal axis. The cannula has a first position that includes the tubes engaged by a first detent means for entry and removal from a patient and a second position that includes the tubes engaged by a second detent means. In the second position, the shield members are flexed to a position transverse to the longitudinal axis and the counter-pressure ring is in direct contact with the patient.

The clear view cannula can include a handle and an advancement mechanism that moves the tubes between the first position and the second position. The advancement mechanism can include threads.

The detents preferably include a protuberance that mates with at least two channels. The clear view cannula can be moved between the first position and second position by a single hand. The first tube can also include the shield structure and the second tube can include apertures through which the shield structure deploys. The second tube can also include the shield structure.

The counter-pressure ring provides visibility to the skin beneath the ring to assess the applied pressure. The counter-pressure ring can be transparent. The counter-pressure ring can also include a pressure sensor and a pressure sensor indicator. The counter-pressure ring can have through holes. The counter-pressure ring includes a proximal extension for manipulation.

### BRIEF DESCRIPTION OF THE DRAWINGS

Preferred embodiments of the invention are described below with reference to the drawings, wherein like numerals are used to refer to the same or similar elements.
FIG. 1 is a top view of a clear view cannula in a first position that includes a first tube and a second tube;
FIG. 2 is a side cross-sectional view taken along lines 2-2 of the cannula of FIG. 1;
FIG. 3 is the side cross-sectional view of the cannula of FIG. 1 in a second position with the shield structure deployed from the second tube;
FIG. 4 is a side cross-sectional view of an embodiment of the cannula of the invention based on FIG. 1 in the first position after entry into a body portion of a patient that includes detents constructed in accordance with the present disclosure;
FIG. 5 is a close-up side cross-sectional view of a distal end portion of the cannula of FIG. 4;
FIG. 6 is a side cross-sectional view of the cannula of FIG. 4 with the shield members deployed;
FIG. 7 is a close-up of a distal end portion of the first tube of the cannula of FIG. 4;
FIG. 8 is a side cross-sectional view of the cannula of FIG. 4 in the second position with the counter-pressure ring in direct contact with the skin of the patient;
FIG. 9 is a top view of another embodiment of the clear view cannula of FIG. 1 in the second position that has the shield structure deployed from the first tube through an aperture in the second tube constructed in accordance with the present disclosure;
FIG. 10 is a side cross-sectional view taken along lines 10-10 of the clear view cannula of FIG. 9;
FIG. 11 is a close-up of the distal end portion of the second tube of the cannula of FIG. 9 that shows the apertures for the shield members;
FIG. 12 is a close-up of a distal end portion of the first tube of the cannula of FIG. 9 that shows the shield members;
FIG. 13 is a cross-sectional view taken along lines 13-13 of the clear view cannula of FIG. 9;
FIG. 14 is a variant of FIG. 1, which is not according to the invention, but shows features that can be used in the invention, including a screw mechanism for the movement of the cannula between the first position and the second position and a counter-pressure ring; and
FIG. 15 is the side cross-sectional view of the cannula of FIG. 14 in the second position with the counter-pressure ring advanced.

### DETAILED DESCRIPTION

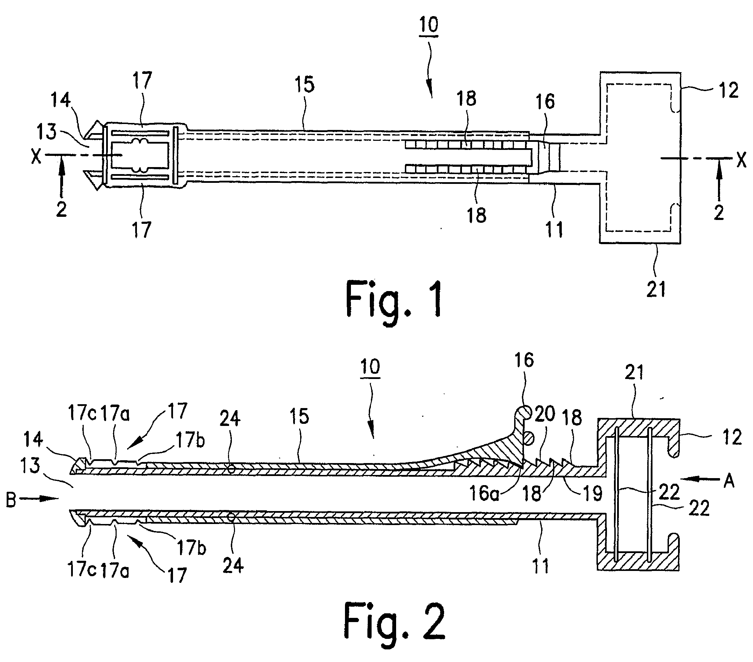

Referring to FIG. 1, a clear view cannula 10 is described which is not according to the invention but is useful for understanding the invention. It includes a first tubular cannula body 11 that has a proximal end portion 12 and a distal end portion 13 that define a passageway that has a central longitudinal axis-X. Cannula 10 is shown in a first position for penetration through a body wall and into a portion of a body such as a joint and/or an anatomical cavity. Proximal end portion 12 includes a handle 21.

A second tube 15 is concentrically mounted and slidably secured with cannula body 11 in an adjoined close fitting relationship. Second tube or cylindrical sleeve 15 has a proximal end that includes a raised shoulder 16 for the positioning of second tube 15 relative to first tube 11. Shoulder 16 preferably extends radially from the vicinity of proximal end portion 12 in the first position of cannula 10. The distal end portion of second tube 15 is preferably connected to distal end portion 13 by a snap-fit detent, but the tubes can be connected by any conventional means such as a spot weld, heat bond and/or a mechanical connection such as a pivot or hinge.

Cannula 10 can be constructed of materials suitable for medical applications such as but not limited to polymers, composites, metals and glass. Cannula 10 is preferably a reusable assembly that can be disassembled and sterilized, but it can also be constructed to be disposable device that is intended for a single use.

A shield structure is included in the distal end portion of second tube 15 in this embodiment and includes a plurality of shield members 17. Shield members 17 are shown in a first position of cannula 10 approximately parallel to the longitudinal axis, closed and can provide a fluid tight seal. Shield members 17 are movable between the first position and a second flexed and deployed position of cannula 10 by a single hand that can simultaneously grasp handle 21 and manipulate shoulder 16 relative to handle 21.

As shown in FIGS. 1 and 2, cannula 10 includes an advancement mechanism for moving between the first position and the second position. In one embodiment, the advancement mechanism includes a plurality of closely spaced apart longitudinally extending teeth members 18 on the outer surface of a tubular wall of first tube 111 in the vicinity of proximal end portion 12. The number and length of the plurality of teeth members 18 is dependent upon a particular application of cannula 10. Teeth members 18 have a face 19 that is preferably approximately perpendicular to the longitudinal axis for the retention of second tube 15 in position relative to first tube 11. A rear portion 20 of each tooth 18 slopes toward proximate end 12. The inner end of shoulder 16 of second tube 15 includes a depending detent 16a that can be positioned to engage one of the faces 19 of the teeth members 18 to preclude the proximal travel of first tube 11 relative to second tube 15. The slope of rear portion 20 facilitates the movement over and/or engagement of teeth 18 by depending detent 16a.

Handle 21 can be readily grasped by a user with one hand and concurrently move second tube 15 in at least the distal and proximal directions along the longitudinal axis with the fingers of the same hand by manipulating shoulder 16. Handle 21 can take any shape that supports moving cannula 10 between the first position and second position using a single hand. Handle 21 can be monolithically formed with cannula 10 or a separate assembly that is removably connected to proximal end portion 12.

Handle 21 includes one or more fluid tight valve members or seals 22 to prevent fluids from entering into or exiting from a joint or an inflated body cavity through the longitudinal aperture of first tube 11 of cannula 10 in the distal direction and proximal direction as shown by arrows A and B, respectively. Valve members 22 can be any type of seal suitable for use in conjunction with minimally invasive surgery. In addition, a seal 24 such as a gasket or O-ring can be seated between the outer surface of the wall of first tube 11 and the inner surface of the wall of second tube 15 to preclude the passage of fluids between the tubes.

Referring now to Fig. 2, shield members 17 are in the first position and preferably approximately parallel to the longitudinal axis. Shield members 17 bend or flex at a midpoint hinge 17a, proximal hinge 17b and a distal hinge 17c. The flexible hinges have a thickness that is less than the thickness of the wall of each shield member 17 to facilitate bending between the first position and a second deployed position. The shield structure advantageously provides shield members 17 with a controlled degree of longitudinal rigidity in the first position, a bias to the first position and flexibility to move between the first position and the second deployed position.

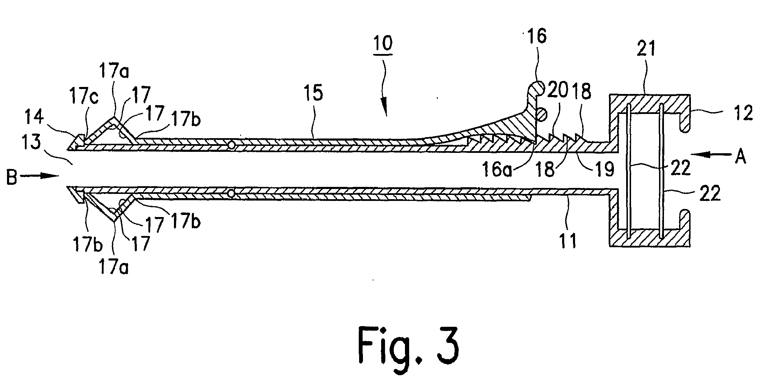

As shown in FIG. 3, shield members 17 are in the second deployed position with midpoint hinge 17a forming an approximately 90 degree angle and hinges 17b and 17c at angles of approximately forty-five degrees from the longitudinal in this one preferred embodiment. Shield members 17 in the second position can be at any position between approximately parallel and approximately perpendicular to the longitudinal axis. Shield members 17 can also vary in longitudinal length, outer surfaces width and radial thickness depending upon their intended application. Shield members 17 are retained in the deployed position by the engagement of depending detent 16a with teeth members 18.

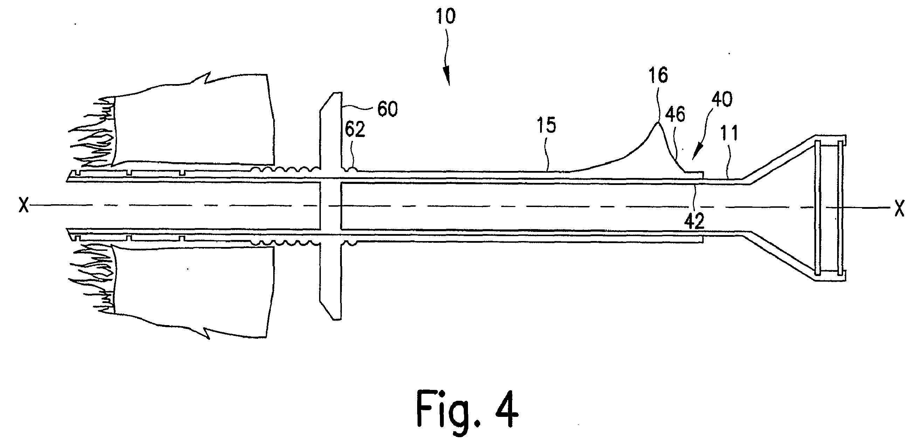

Referring now to Fig. 4, clear view cannula 10 in the preferred embodiment includes a system of detents 40 that limit the relative movement along the longitudinal axis and define preset positions for tubes 11 and 15. Detents 40 advantageously provide the user of cannula 10 with positions that define one or more stages of deployment of shield members 17 and the first position for entry into or removal from the patient. In addition, detents 40 can limit the angular flexing of shield members 17 to advantageously control the bending stresses on each flexible hinge 17a, 17b and 17c to a desired predetermined level and thereby prolong the operational lifespan of each clear view cannula 10. Cannula 10 in this preferred embodiment also includes a counter-pressure ring 60 that moves with and independent of tube 15.

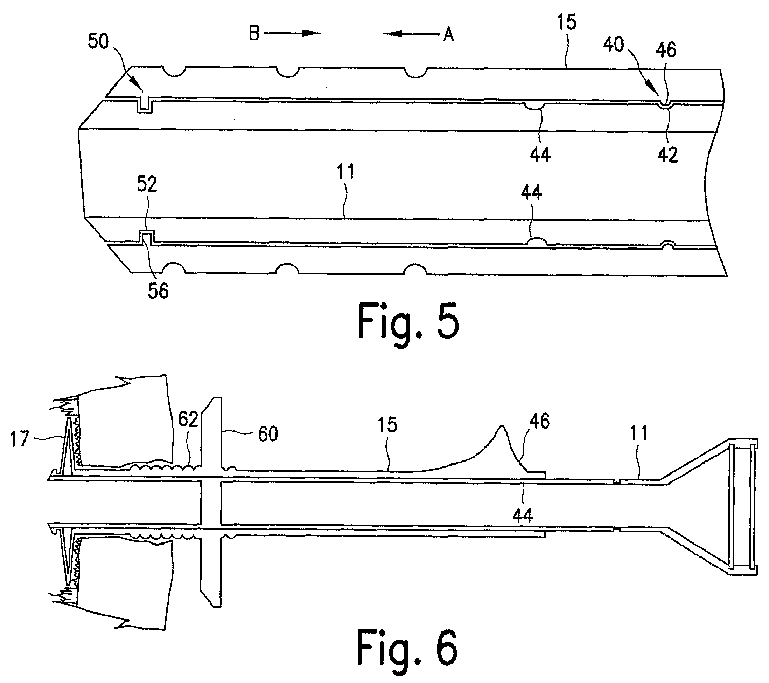

As shown in Figs. 4 and 5 and continuing with the preferred embodiment, detents 40 have a flexible snap-fit relationship that includes at least two channels 42 and 44 on the outer surface of first tube 11 at predetermined distances along the longitudinal axis. Second tube 15 has at least one protuberance 46 at a predetermined position on the longitudinal axis. Protuberance 46 mates with channel 42 to cooperatively define the first position and channel 44 for the second position of cannula 10. Tubes 11 and 15 are sufficiently flexible to accommodate the increase and/or decrease in diameter required by protuberance 46 in direct contact with the outer surface of the interfacing adjoined tube when positioned between channels 42 and 44.

Referring now to Figs. 2, 5 and 6, cannula 10 is in the second position with second tube 15 moved distally relative to first tube 11. The second position of cannula 10 includes protuberance 46 engaged by distal channel 44 and shield members 17 moved from the first position to the expanded position. As shown in this one preferred embodiment, hinges 17b, 17c and shield members 17 are approaching the perpendicular to the longitudinal axis. Channels 42, 44 and mating protuberance 46 can have any corresponding geometrical shape for mating such as concave and convex, angular or flexible elements. Channel 42, channel 44 and mating protuberance 46 can also be segmented or continuous elements that extend around the circumference of first tube 11 and corresponding respective inside surface of second tube 15.

The sidewalls of channels 42, 44 and mating protuberance 46 can be symmetrical or asymmetrical depending upon the desired application of a given detent 40. The sidewalls can provide stops or limits of travel in one direction along the longitudinal axis, a fixed position as well as the ease of entry into or exit from a given position for a desired direction of movement. In the preferred embodiment, for example, distal channel 44 includes a distal sidewall that engages the distal sidewall of protuberance 46 and precludes any further distal travel of tube 15 relative to tube 11 in the direction of arrow A. Similarly, the proximal sidewall of channel 44 is sloped to engage the proximal sidewall of protuberance 46 to retain tube 15 in the second position of cannula 10 against the bias of shield members 17 and restrict the proximal movement of tube 15 relative to tube 11. The application of a predetermined amount of proximally directed force, in the direction shown by arrow B, can overcome the engagement of protuberance 46 with channel 44.

In the preferred embodiment, detents 40 define the proximal and distal limits of travel of second tube 15 relative to first tube 11 and can augment or replace teeth members 18 and depending detent 16a. The distance between channels 42 and 44 preferably defines the maximum angle of deployment of shield members 17 in the second position and the approximate alignment of shield members 17 in the first position. For example, a less than perpendicular angle for shield members 17 can increase the volume of surgical work area and reduce the level of stress of hinge 17a, 17b and 17c. Detents 40 include a protuberance 46 on first tube 11 that mates with channels 42 and 44 on second tube 15. The mating of protuberance 46 and channels 42 and 44 can also include a tactile and/or an aural indication.

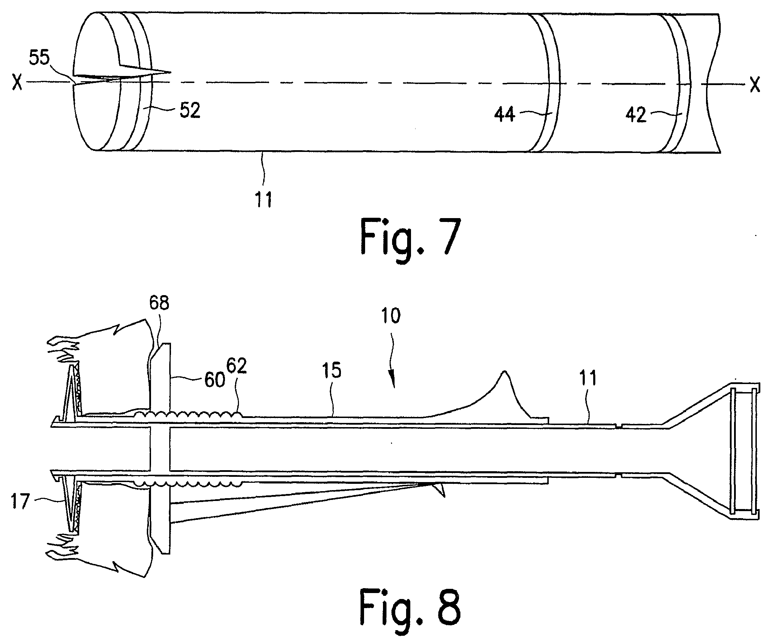

As shown in Figs. 5 and 7, tube 11 can also include one or more detents 50 that securely connect first tube 11 and second tube 15. Detent 50 in this preferred embodiment connects the distal end portion 13 of first tube 11 and the distal end portion of second tube 15. Detent 50 remains fixed during the movement of cannula 10 between the first position and second position. Channel 52 has sidewalls that engage and lock with the sidewalls of protuberance 56. In this embodiment, the sidewalls of channel 52 and protuberance 56 are shown as perpendicular to the longitudinal axis, but the sidewalls can have any shapes that combine to engage and lock tubes 11 and 15 together.

Distal end portion 13 of first tube 11 preferably defines an aperture or slot 55 that extends from a distal edge proximally a predetermined distance. Slot 55 accommodates the flexing of distal end 13 during the assembly and/or positioning of protuberance 56 in channel 52. Detent 50 can be a fixed permanent or a detachable connection. Tubes 11 and 15 of cannula 10 can advantageously be assembled using detent 50 without the use of adhesives or other fasteners. Detent 50 as a detachable connection can be readily disassembled, as required, for sterilization.

As shown in FIG. 8, counter-pressure ring 60 is selectively longitudinally positionable on a plurality of ribs 62 on second tube 15. Counter-pressure ring 60 has an annular plate shape with a proximally directed planar face, a distally directed planar face, an inner circular surface that engages with ribs 62 and an outer circumference surface 68. A slot extends from inner surface to outer surface 68. Counter-pressure ring 60 is preferably made of a material with sufficient structural integrity to function as a counter-pressure to the deployed shield members 17. Ring 60 can also include a proximal extension that enables the distal and proximal positioning of ring 60 using a single hand that is simultaneously grasping handle 21.

Counter-pressure ring 60 can indicate and/or assess the amount of pressure applied to the patient's skin. In one preferred embodiment, counter-pressure ring 60 is at least partially fabricated of a transparent material such that a visual inspection can be made of the skin of the patient during the surgical procedure. Alternative visual indications can be provided by through holes in ring 60 that allow direct visibility to the skin. Ring 60 can also include one or more pressure sensors and indicators that measure the amount of applied pressure. The planar proximal of ring 60 is preferably tapered in the vicinity of outer edge 68 to minimize the stress that is applied to the body of the patient.

Ribs 62 have a larger diameter than inner surface and securely position ring 60 for the application of a distally directed counter-pressure to the patient. The inner surface of ring 60 can expand or open up using the split or slot between surface and surface 68 for movement along the longitudinal axis of second tube 15 over the peaks of ribs 62. Ring is biased to return to the initial closed position after expansion. The slot can also include an interface that provides a fluid tight seal such as tongue and groove, spiral overlap or other engaging connection that can expand and return to the initial position. It is understood that ribs 62 and inner surface can include other forms of engagement for the application of pressure and movement besides slot such as a segmented rib, slots or threads that can adjust and fix the position of ring 60 for the application of counter-pressure.

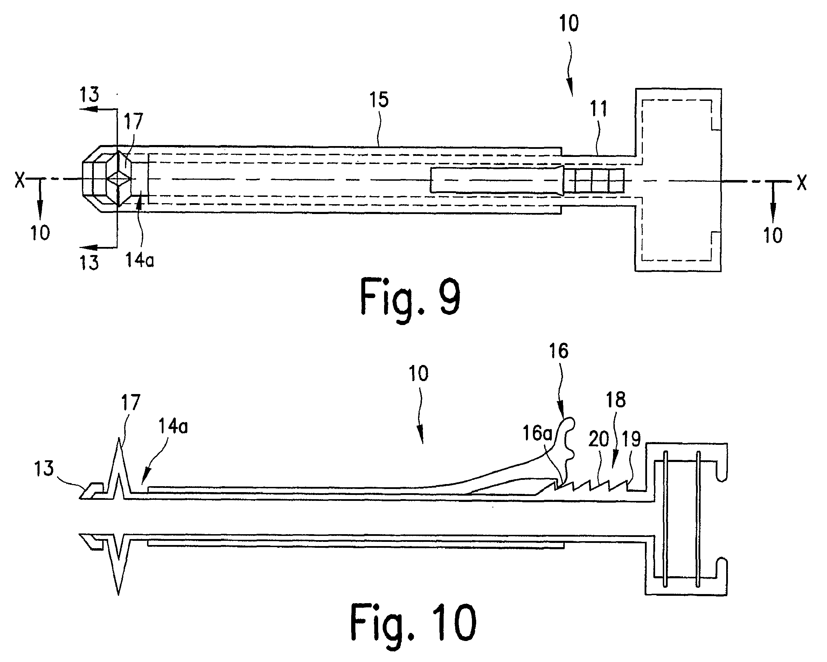

FIGS. 9 and 10 show a cannula 10 which does not illustrate all the features of the invention, but shows features that can be used with the invention. First tube 11 includes the shield structure with a plurality of shield members 17 and the distal end portion 13 of second tube 15 defines a plurality of apertures 14a. Apertures 14a are aligned with and dimensionally accommodate the deployment of shield members 17. Shield members 17 are preferably approximately parallel with the longitudinal axis-X in the first position and move through apertures 14a as first tube 11 moves relative to second tube 15 between the first position and second deployed position. Shield members can deploy up to approximately perpendicular to the longitudinal axis.

Cannula 10 in this embodiment has teeth 18 that have faces 19 that are approximately perpendicular to the longitudinal axis. Front portions 20 slope downward in the distal direction. Teeth 18 and depending detent 16a accommodate the ease of movement of tube 11 in the distal direction relative to tube 15 and fix tube 11 in a desired position against the bias in shield members 17 similar to the embodiment of FIG. 1.

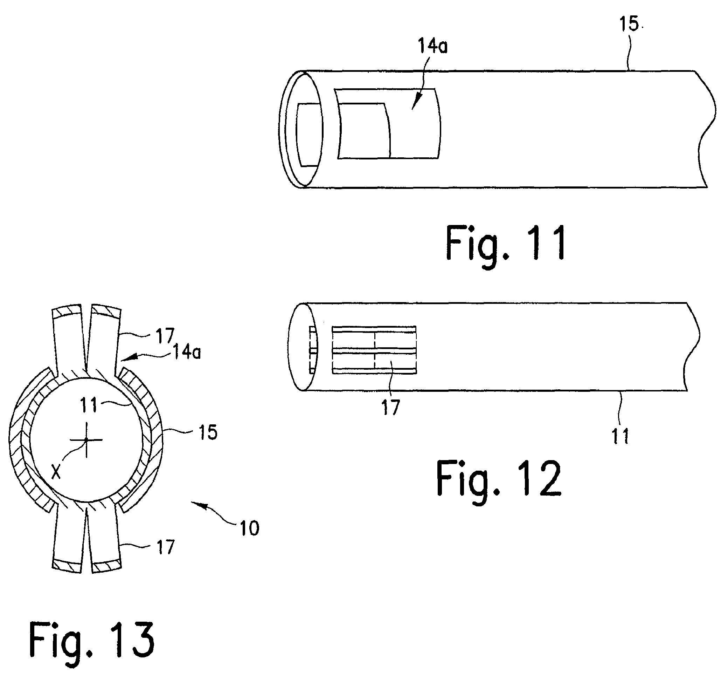

As shown in FIGS. 11 and 12 and continuing with this embodiment, tube 15 has two diametrically opposed apertures 14a, but cannula 10 can include any positional relationship of two or more apertures 14a and corresponding shield members 17 that provide the required sealing and support when positioned in the body of the patient. In one embodiment, apertures 14a are defined by edges or portions of sidewalls in second tube 15 that are tapered inwardly to provide a smooth transition and minimize trauma to the adjoining tissue during penetration and withdrawal of cannula 10 from the patient's body. Shield members 17 in the first position can also have an outer surface that is flush with the outer surface of the tubular wall of second tube 15.

Referring now to FIG. 13, cannula 10 is shown in this embodiment with first tube 11 and second tube 15 concentrically aligned about longitudinal axis-X. Shield members 17 on first tube 11 are deployed in the second position through aperture 14a of second tube 15.

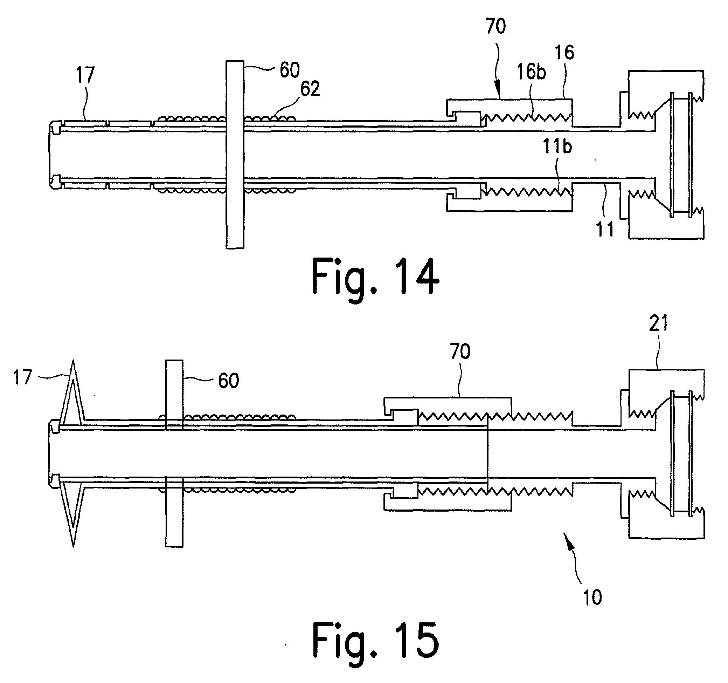

FIG. 14 shows a cannula 10 which does not illustrate all the features of the invention, but shows features that can be used with the invention. In this embodiment, cannula 10 has an advancement mechanism for movement between the first and second positions that includes a threaded actuator 70. Actuator 70 preferably includes a shoulder 16 that is rotatingly connected to second tube 15. Shoulder 16 also includes threads 16b that connect with threads 11b of first tube 11. The rotation of threaded actuator 70 in a first direction advances shoulder 16 and second tube 15 distally to deploy and/or flex shield members 17 from the first position to the second position of cannula 10. Counter-pressure ring 60 advances in position on ribs 62 with the distal movement of tube 15. The rotation of threaded actuator 70 in the opposed second direction withdraws shoulder 16 proximally and moves shield members 17 and counter-pressure ring 60 in the direction of the first position.

Referring now to FIG. 15, cannula 10 is shown in the second position with shield members 17 deployed and counter-pressure ring 60 forwarded to be in apposition the patient's body. As required, ring 60 is then placed directly in contact with the patient. In this embodiment the distal advancement of actuator 70 deploys shield members 17 and positions counter-pressure ring 60 approximately in apposition with the patient's body. Actuator 70 is turned in a second direction to return shield members 17 to the first position and cannula 10. In this embodiment, handle 21 is threaded to tube 11 and can be separately removed from cannula 10.

Referring now to FIG. 4, cannula 10 is shown in operation in the first position after having gained entry into a joint. The first position of cannula 10 is defined in this embodiment by the engagement of protuberance 46 of tube 15 with channel 42 of proximally positioned detent 40. Counter-pressure ring 60 is in a predetermined position on ribs 62 of second tube 15.

As shown in FIG. 6, when tube 15 is positioned distally such that protuberance 46 mates with distal detent 40 of tube 11, shield structure 17 is moved from the first to the second position of cannula 10 and counter-pressure ring 60 moves into apposition with the patient. As required, counter-pressure ring 60 can manually be adjusted to be in direct contact with, increase pressure on or decrease pressure on the patient. The longitudinal movement of counter-pressure ring 60 across ribs 62 is made by forcing inner annular surface over the peaks of ribs 62. The slot expands to accommodate the increased diameter of annular surface 62 to overcome the diameter of the peaks of ribs 62. Counter-pressure ring 60 has sufficient elasticity to return to the initial position when repositioned in a trough between ribs 62.

Counter-pressure ring 60 provides an adjustable and approximately distally directed force that opposes the proximally directed force applied by shield structure 17. Ring 60 is thus a stabilizing structure for cannula 10 in the second position that retains cannula at the desired angular relationship with the patient. In one preferred embodiment of ring 60 the at least partially transparent material of construction and/or through holes provide a visual indication of the amount of pressure applied against the skin of the patient and/or between shield members 17. The visual indication of the applied pressure enables the viewing of any change in the skin color that is beneath and/or in contact with ring 60 that can indicate excessive pressure and/or the start of necrosis. Alternatively, ring 60 can include a pressure sensor and indicator that can be set to indicate or warm when an excessive amount of pressure is applied by ring 60.

Referring now to FIG. 8, cannula 10 in the second position includes shield members 17 deployed and counter-pressure ring 60 in direct contact with the patient. Counter-pressure ring 60 stabilizes the angular relationship between cannula 10 and the patient's body. The combination of deployed shield members 17 and ring 60 can also provide a fluid tight seal between the cannula and the patient's body and accommodate the pulling back on the cannula during surgical procedures without breaking the seal in order to provide increased visibility within the joint or body cavity of the patient.

Cannula 10 is returned to the first position by moving second tube 15 proximally relative to first tube 11. In this preferred embodiment, counter-pressure ring 60 is simultaneously repositioned proximally with ribs 62 of second tube 15.

In the preceding specification, the invention has been described with reference to specific exemplary embodiments thereof. It will be evident, however, that various modifications, combinations and changes may be made thereto without departing from the broader scope of the invention as set forth in the claims that follow. For example, the relative movement between the tubes or of one tube relative to the adjoined tube includes any combination of movements of the two tubes to affect the movement between the first and second positions. In addition, the movement of one tube can be provided by one or more combinations of the advancement mechanisms of engaging teeth and threads with detents. The specification and drawings are to be regarded in an illustrative manner rather than a restrictive sense.

## Claims

1. A clear view cannula (10) for use by a single hand in arthroscopic surgical operations that comprises:
a first tube (11) that includes a distal end portion (13) and a proximal end portion (12) that define a central longitudinal axis, the tube (11) has a tubular wall that defines an aperture aligned with the longitudinal axis, the proximal end portion (12) includes a handle (21);
a second tube (15) that includes a distal end portion and a proximal end portion, the second tube (15) positioned external to and adjoins the first tube (11), the second tube (15) has a tubular wall that defines an aperture aligned with the longitudinal axis, the distal end portions of the tubes (11) and (15) being connected, the second tube (15) includes a plurality of ribs (62) separated by troughs;
a shield structure that includes a plurality of shield members (17) positioned in the tubular wall of the distal end portion of the second tube (15), the second tube (15) longitudinally movable relative to the first tube (11);
a tapered tip positioned on the distal end portion of one of the tubes and a shoulder (16) positioned on the second tube (15) to facilitate the relative movement between the tubes;
a counter-pressure ring (60) movably connected to the second tube (15), the counter-pressure ring (60) being for providing an indication of the amount of counter-pressure applied against the skin of a patient to include the indication of the application of excessive pressure by the counter-pressure ring to the tissue of a patient;
the counter-pressure ring (60) has an annular plate shape that includes a distally directed planar face
and
a first position in which the first tube (11) engages with the second tube (15) by a first detent (40) and a second position in which the first tube (11) engages with the second tube (15) by a second detent (50), wherein, in the second position, the second tube (15) is repositioned distally along the longitudinal axis relative to the first tube (11) and the shield structure is deployed, the counter-pressure ring (60) in the second position being adjustable along the longitudinal axis and adjustable to stabilize the angular position of the cannula (10), the detents (40, 50) limiting the distal position and the proximal position of the second tube (15) and the flexing of the shield structure relative to the first tube (11), the counter-pressure ring (60) distally directed planar face includes a fluid tight external seal in combination with the deployed shield structure in the second position and the counter-pressure ring (60) provides a fluid tight seal between the second tube (15) and the counter-pressure ring (60) in the second position,
**characterized in that**:
the counter-pressure ring (60) includes a split, the split extends between an inner annular surface of the counter-pressure ring (60) and an outer surface diameter of the counter-pressure ring (60), the inner annular surface of the counter-pressure ring (60) expands and the split opens for the longitudinal movement of the counter-pressure ring (60) over at least one of the plurality of ribs (62), the split biased to a closed position and the split being closed and sealed in the second position.

2. The clear view cannula of claim 1, wherein an inside surface of the second tube (15) tubular wall includes protuberances (46) and the outside surface of the tubular wall of the first tube (11) includes channels (42, 44), the arrangement of protuberances (46) and channels (42, 44) forming the detents (40, 50) that define the first and second positions.

3. The clear view cannula of claim 2 wherein the protuberances (46) mate with the channels (42, 44) and the mating of the protuberances (46) with the channels (42, 44) provide an aural indication of the mating.

4. The clear view cannula of claim 1, wherein the protuberances (46) mate with the channels (42, 44) and the mating of the protuberances (46) with the channels (42, 44) provide a tactile indication of the mating.

5. The clear view cannula of claim 1, wherein the first tube (11) distal end portion (13) defines an aperture (55) in the tubular wall that extends from a distal edge proximally a predetermined distance, first tube (11) distal end portion (13) includes a further channel (52) that engages a protuberance (56) of the second tube (15) distal end portion, the engagement of the protuberance (56) and further channel (52) fixes the distal end of the cannula at the engagement of the protuberance (56) and further channel (52) the aperture (55) in the tubular wall accommodates the flexing of distal end portion (13) during the assembly of the first tube (11) and second tube (15) of the cannula (10).

6. The clear view cannula of claim 1, wherein the plurality of shield members (17) in the first position is approximately parallel to the longitudinal axis and a fluid tight seal with the tubular wall of the second tube.

7. The clear view cannula of claim 5, wherein the engagement of the further channel (52) and protuberance (56) is a snap fit connection between first tube (11) and second tube (15).

8. The clear view cannula of claim 1, wherein the counter-pressure ring (60) positioned in a trough between two ribs (62) has sufficient elasticity to return to the second position.

## Patentansprüche

1. Klarsichtkanüle (10) zur Verwendung mit einer einzelnen Hand in arthroskopischen chirurgischen Operationen, welche umfasst:
ein erstes Rohr (11), welches einen distalen Endabschnitt (13) und einen proximalen Endabschnitt (12) umfasst, welche eine zentrale Längsachse definieren, wobei das Rohr (11) eine rohrförmige Wand aufweist, welche eine Öffnung definiert, welche mit der Längsachse ausgerichtet ist, wobei der proximale Endabschnitt (12) eine Handhabe (21) umfasst;
ein zweites Rohr (15), welches einen distalen Endabschnitt und einen proximalen Endabschnitt umfasst, wobei das zweite Rohr (15) außerhalb des und am ersten Rohr (11) anliegend angeordnet ist, wobei das zweite Rohr (15) eine rohrförmige Wand aufweist, welche eine Öffnung definiert, welche mit der Längsachse ausgerichtet ist, wobei die distalen Endabschnitte der Rohre (11) und (15) verbunden sind, wobei das zweite Rohr (15) eine Mehrzahl von Stegen (62) umfasst, welche von Vertiefungen geteilt sind;
eine Schirmstruktur, welche eine Mehrzahl von Schildelementen (17) umfasst, welche in der Rohrwand des distalen Endabschnitts des zweiten Rohrs (15) angeordnet sind, wobei das zweite Rohr (15) relativ zum ersten Rohr (11) längsbeweglich ist;
eine verjüngte Spitze, welche auf dem distalen Endabschnitts eines der Rohre angeordnet ist, und eine Schulter (16), welche auf dem zweiten Rohr (15) angeordnet ist, um di relative Bewegung zwischen den Rohren zu ermöglichen;
einen Gegendruckring (60), welcher mit dem zweiten Rohr (15) beweglich verbunden ist, wobei der Gegendruckring (60) als Anzeige für den Wert des Gegendrucks, welcher gegen die Haut eines Patienten geübt wird, dient, um die Anzeige eines Überdrucks durch den Gegendruckring gegen das Gewebe eines Patienten zu enthalten;
wobei der Gegendruckring (60) die Form einer Ringplatte aufweist, welche eine distal gerichtete plane Fläche umfasst,
und
eine erste Stellung, in der das erste Rohr (11) in das zweite Rohr (15) mittels einer ersten Klinke (40) eingreift, und eine zweite Position, in der das erste Rohr (11) in das zweite Rohr (15) mittels einer zweiten Klinke (50) eingreift, wobei, in der zweiten Stellung, das zweite Rohr (15) distal entlang der Längsachse relativ zum ersten Rohr (11) umpositioniert wird, und die Schildstruktur ist entfaltet, wobei der Gegendruckring (60) in der zweiten Stellung entlang der Längsachse, und zum Stabilisieren der Winkelstellung der Kanüle (10) verstellbar ist, wobei die Klinken (40, 50) die distale Position und die proximale Position des zweiten Rohrs (15) begrenzen, sowie das Verbiegen der Schildstruktur relativ zum ersten Rohr (11), wobei die distal gerichtete plane Fläche des Gegendruckrings (60) eine fluiddichte äußere Abdichtung umfasst, in Kombination mit der entfalteten Schildstruktur in der zweiten Stellung und der Gegendruckring (60) eine fluiddichte Abdichtung zwischen dem zweiten Rohr (15) und dem Gegendruckring (60) in der zweiten Stellung bereitstellt,
**dadurch gekennzeichnet, dass**
der Gegendruckring (60) einen Spalt umfasst, wobei der Spalt sich zwischen einer inneren Ringfläche des Gegendruckrings (60) und einem Durchmesser der Außenfläche des Gegendruckrings (60) erstreckt, wobei die innere Ringfläche des Gegendruckrings (60) sich ausweitet und der Spalt öffnet sich, um eine Längsbewegung des Gegendruckrings (60) über zumindest einen der Mehrzahl von Stegen (62) bereitzustellen, wobei der Spalt in eine geschlossene Stellung gedrückt wird und der Spalt in der zweiten Stellung geschlossen und abgedichtet ist.

2. Klarsichtkanüle nach Anspruch 1, wobei eine innere Oberfläche der Rohrwand des zweiten Rohrs (15) Vorsprünge (46) und die äußere Oberfläche der Rohrwand des ersten Rohrs (11) Kanäle (42, 44) umfasst, wobei die Anordnung von Vorsprüngen (46) und Kanälen (42, 44) die Klinken (40, 50) bildet, welche die erste und die zweite Stellung definieren.

3. Klarsichtkanüle nach Anspruch 2, wobei die Vorsprünge (46) mit den Kanälen (42, 44) koppeln und das Koppeln der Vorsprünge (46) mit den Kanälen (42, 44) eine hörbare Anzeige des Koppelns liefert.

4. Klarsichtkanüle nach Anspruch 1, wobei die Vorsprünge (46) mit den Kanälen (42, 44) koppeln, und das Koppeln der Vorsprünge (46) mit den Kanälen (42, 44) eine haptische Anzeige des Koppelns liefert.

5. Klarsichtkanüle nach Anspruch 1, wobei der distale Endabschnitt (13) des ersten Rohrs (11) eine Öffnung (55) in der Rohrwand definiert, welche sich von einem distalen Rand, in proximaler Richtung um einen gegebenen Abstand erstreckt, wobei der distale Endabschnitt (13) des ersten Rohrs (11) einen weiteren Kanal (52) umfasst, welcher in einen Vorsprung (56) des distalen Endabschnitts des zweiten Rohrs (15) eingreift, wobei der Eingriff zwischen dem Vorsprung (56) und dem weiteren Kanal (52) das distale Ende der Kanüle beim Koppeln zwischen Vorsprung (56) und weiterem Kanal(52) fixiert, wobei die Öffnung (55) in der Rohrwand das Biegen des distalen Endabschnitts (13) während des Zusammenbau des ersten (11) und des zweiten (15) Rohrs der Kanüle (10) aufnimmt.

6. Klarsichtkanüle nach Anspruch 1, wobei die Mehrzahl der Schildelemente (17) in der ersten Stellung ungefähr parallel zur Längsachse angeordnet ist und eine fluiddichte Abdichtung mit der Rohrwand des zweiten Rohrs bildet.

7. Klarsichtkanüle nach Anspruch 5, wobei der Eingriff zwischen dem weiteren Kanal (52) und dem Vorsprung (56) eine Schnappverbindung zwischen dem ersten Rohr (11) und dem zweiten Rohr (15) ist.

8. Klarsichtkanüle nach Anspruch 1, wobei der Gegendruckring (60), welcher in einer Vertiefung zwischen zwei Stege (62) angeordnet ist, eine ausreichende Elastizität aufweist, um in die zweite Stellung zurückzukehren.

## Revendications

1. Canule à bonne visibilité (100) pour une utilisation par une seule main dans des opérations chirurgicales arthroscopiques, comprenant :
un premier tube (11), englobant une partie d'extrémité distale (13) et une partie d'extrémité proximale (12) définissant un axe longitudinal central, le tube (11) comportant une paroi tubulaire définissant une ouverture alignée avec l'axe longitudinal, la partie d'extrémité proximale (12) englobant une poignée (21) ;
un deuxième tube (15), englobant une partie d'extrémité distale et une partie d'extrémité proximale, le deuxième tube (15) étant positionné à l'extérieur du premier tube (11) et étant adjacent à celui-ci, le deuxième tube (15) comportant une paroi tubulaire définissant une ouverture alignée avec l'axe longitudinal, les parties d'extrémité distale des tubes (11) et (15) étant connectées, le deuxième tube (15) englobant plusieurs nervures (62) séparées par des creux ;
une structure de protection, englobant plusieurs éléments de protection (17) positionnés dans la paroi tubulaire de la partie d'extrémité distale du deuxième tube (15), le deuxième tube (15) pouvant être déplacé longitudinalement par rapport au premier tube (11);
une pointe effilée positionnée sur la partie d'extrémité distale de l'un des tubes et un épaulement (16) positionné sur le deuxième tube (15) pour faciliter le mouvement relatif entre les tubes, une bague de contre-pression (60), connectée de manière mobile au deuxième tube (15), la bague de contre-pression (60) servant à fournir une indication de la quantité de contre-pression appliquée contre la peau d'un patient pour inclure l'indication de l'application d'une pression excessive par la bague de contre-pression sur les tissus d'un patient; la bague de contre-pression (60) ayant une forme de plaque annulaire engobant une face plane à direction distale ; et
une première position, dans laquelle le premier tube (11) s'engage dans le deuxième tube (15) par un premier cliquet (40), et une deuxième position, dans laquelle le premier tube (11) s'engage dans le deuxième tube (15) par un deuxième cliquet (50), dans lequel, dans la deuxième position, le deuxième tube (15) est repositionné dans une direction distale le long de l'axe longitudinal par rapport au premier tube (11), et la structure de protection est déployée, la bague de contre-pression (60) pouvant être ajustée dans la deuxième position le long de l'axe longitudinal et pouvant être ajustée pour stabiliser la position angulaire de la canule (10), les cliquets (40, 50) limitant la position distale et la position proximale du deuxième tube (15) et le fléchissement de la structure de protection par rapport au premier tube (11), la face plane à direction distale de la bague de contre-pression (60) englobant un joint d'étanchéité externe étanche au fluide, en combinaison avec la structure de protection déployée dans la deuxième position, et la bague de contre-pression (60) établissant un joint d'étanchéité étanche au fluide entre le deuxième tube (15) et la bague de contre-pression (60) dans la deuxième position ;
**caractérisée en ce que** :
la bague de contre-pression (60) englobe une fente, la fente s'étendant entre une surface annulaire interne de la bague de contre-pression (60) et un diamètre de surface externe de la bague de contre-pression (60), la surface annulaire interne de la bague de contre-pression (60) se dilatant et la fente étant ouverte en vue du déplacement longitudinal de la bague de contre-pression (60) au-dessus d'au moins une des plusieurs nervures (62), la fente étant poussée vers une position fermée et la fente étant fermée et scellée dans la deuxième position.

2. Canule à bonne visibilité selon la revendication 1, dans laquelle une surface interne de la paroi tubulaire du deuxième tube (15) englobe des protubérances (56), la surface externe de la paroi tubulaire du premier tube (11) englobant des canaux (42, 44), l'agencement des protubérances (46) et des canaux (42, 44) formant les cliquets (40, 50) définissant les première et deuxième positions.

3. Canule à bonne visibilité selon la revendication 2, dans laquelle les protubérances (46) sont accouplées avec les canaux (42, 44), l'accouplement des protubérances (46) avec les canaux (42, 44) fournissant une indication auditive de l'accouplement.

4. Canule à bonne visibilité selon la revendication 1, dans laquelle les protubérances (46) sont accouplées avec les canaux (42, 44), l'accouplement des protubérances (46) avec les canaux (42, 44) fournissant une indication tactile de l'accouplement.

5. Canule à bonne visibilité selon la revendication 1, dans laquelle la partie d'extrémité distale (13) du premier tube (11) définit une ouverture (55) dans la paroi tubulaire, s'étendant d'un bord distal dans une direction proximale sur une distance prédéterminée, la partie d'extrémité distale (13) du premier tube (11) englobant un canal additionnel (52) s'engageant dans une protubérance (56) de la partie d'extrémité distale du deuxième tube (15), l'engagement de la protubérance (56) dans le canal additionnel (52) fixant l'extrémité distale de la canule au niveau de l'engagement de la protubérance (56) dans le canal additionnel (52), l'ouverture (55) dans la paroi tubulaire permettant le fléchissement de la partie d'extrémité distale (13) au cours de l'assemblage du premier tube (11) et du deuxième tube (15) de la canule (10).

6. Canule à bonne visibilité selon la revendication 1, dans laquelle les plusieurs éléments de protection (17) dans la première position sont sensiblement parallèles à l'axe longitudinal et à un joint d'étanchéité étanche au fluide du deuxième tube.

7. Canule à bonne visibilité selon la revendication 5, dans laquelle l'engagement du canal additionnel (52) dans la protubérance (56) constitue une connexion à encliquetage entre le premier tube (11) et le deuxième tube (15).

8. Canule à bonne visibilité selon al revendication 1, dans laquelle la bague de contre-pression (60) positionnée dans un creux entre deux nervures (62) présente une élasticité suffisante pour retourner vers la deuxième position.
